(19) Europäisches Patentamt
European Patent Office
Office européen des brevets

(11) **EP 2 941 631 B1**

(12) **FASCICULE DE BREVET EUROPEEN**

(45) Date de publication et mention
de la délivrance du brevet:
**08.11.2017 Bulletin 2017/45**

(21) Numéro de dépôt: **13827027.7**

(22) Date de dépôt: **30.12.2013**

(51) Int Cl.:
*G01N 21/31* (2006.01)    *G01N 21/3504* (2014.01)
*G01N 33/00* (2006.01)

(86) Numéro de dépôt international:
**PCT/FR2013/053281**

(87) Numéro de publication internationale:
**WO 2014/106717 (10.07.2014 Gazette 2014/28)**

(54) **CAPTEUR DE CONCENTRATION DE CO2**

CO2 KONZENTRATIONSSENSOR

CO2 CONCENTRATION SENSOR

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **02.01.2013 FR 1350014**

(43) Date de publication de la demande:
**11.11.2015 Bulletin 2015/46**

(73) Titulaire: **Withings**
**92130 Issy Les Moulineaux (FR)**

(72) Inventeurs:
 • **HUTCHINGS, Cédric**
  **F-92130 Issy Les Moulineaux (FR)**
 • **BUARD, Nadine**
  **F-92190 Meudon (FR)**

 • **CAMPO, David**
  **F-75015 Paris (FR)**
 • **BRAC DE LA PERRIERE, Brice**
  **F-75007 Paris (FR)**
 • **BARROCHIN, Pierre**
  **F-92210 Saint Cloud (FR)**
 • **DEBREUIL, Xavier**
  **F-92130 Issy Les Moulineaux (FR)**

(74) Mandataire: **Cabinet Plasseraud**
 **66, rue de la Chaussée d'Antin**
 **75440 Paris Cedex 09 (FR)**

(56) Documents cités:
 **EP-A2- 2 293 043     JP-A- 2000 249 651**
 **US-A- 5 341 214     US-A1- 2005 173 635**

EP 2 941 631 B1

**Description**

La présente invention est relative aux dispositifs de mesure de concentration d'un gaz prédéterminé, en particulier le CO2.

Plus particulièrement, l'invention concerne un dispositif de mesure de la concentration d'un gaz prédéterminé, comprenant une cavité renfermant du gaz prédéterminé dans une concentration à mesurer, une source de lumière apte à émettre des rayons lumineux notamment infrarouge dans la cavité, un détecteur infrarouge configuré pour recevoir une partie des rayons lumineux infrarouge dans une gamme spectrale prédéfinie correspondant à une forte absorption du gaz prédéterminé.

Il est connu dans l'art antérieur du document US8003944 de placer un second détecteur infrarouge pour créer un canal de référence qui permet de s'affranchir des dérives lentes liées par exemple au vieillissement de la lampe. Les détecteurs en question sont onéreux et volumineux. De plus, leur consommation électrique est un inconvénient. Le document US 2005/0173635 A1 décrit un dispositif de mesure d'un gaz prédéterminé, notamment du CO2, tel que défini dans le préambule de la revendication 1. Il est donc apparu un besoin d'optimiser ce genre de dispositif pour en diminuer le coût et l'encombrement, sans pour pour autant diminuer leurs performances et leur précision.

A cet effet, l'invention propose notamment un dispositif de mesure de la concentration d'un gaz prédéterminé, en particulier du CO2, comprenant :

- une cavité renfermant un ensemble gazeux contenant du gaz prédéterminé dans une concentration à mesurer,
- une source de lumière, de type lampe à filament ou diode électroluminescente, apte à émettre dans la cavité des rayons lumineux dans une gamme spectrale de base incluant du visible et de l'infra-rouge,
- un détecteur infrarouge configuré pour recevoir une partie des rayons lumineux dans une première gamme spectrale prédéfinie correspondant à une forte absorption du gaz prédéterminé, et pour transformer une puissance reçue en signal électrique
- une photodiode configurée pour recevoir une partie des rayons lumineux dans une deuxième gamme spectrale prédéfinie correspondant à une faible absorption du gaz prédéterminé,
- une unité de commande configurée pour calculer la concentration du gaz prédéterminé en comparant un signal électrique représentatif de la puissance lumineuse reçue par le détecteur infrarouge à un signal électrique représentatif de la puissance lumineuse reçue par la photodiode, dans lequel la photodiode travaille dans le domaine visible, principalement sensible aux longueurs d'onde comprises entre 0,4 et 0,8 $\mu$m. Grâce à ces dispositions, on utilise un seul détecteur de type thermopile, et on dispose néanmoins d'un canal de référence permettant d'éliminer du calcul les dérives rapides ou lentes du dispositif comme les défauts de jeunesse ou le vieillissement de la source lumineuse, la présence de condensation dans la cavité, l'encrassement de la cavité, etc...

En outre, on utilise une photodiode peu coûteuse moyennant quoi le prix du capteur de CO2 est attractif.

Dans des modes de réalisation du procédé selon l'invention, on peut éventuellement avoir recours en outre à l'une et/ou à l'autre des dispositions suivantes, prises isolément ou en combinaison :

- le gaz prédéterminé peut-être particulièrement du dioxyde de carbone CO2 et l'ensemble gazeux est l'air ambiant ; de sorte qu'on peut mesurer la concentration de CO2 dans l'air ambiant ;
- le dispositif peut comprendre un filtre interposé entre la source et le détecteur, ledit filtre étant centré sur la longueur d'onde $\lambda 1 = 4,26$ $\mu$m; ainsi on peut mesurer principalement l'atténuation provoquée par le CO2 et éliminer d'autres phénomènes d'atténuation ;
- la cavité peut avoir une forme générale tubulaire, ayant de préférence un diamètre inférieur à 10 mm et une longueur de préférence inférieure 10 cm, les rayons étant dirigés substantiellement selon une direction axiale X de la forme tubulaire ; moyennant quoi un tel dispositif peut être intégré dans un objet aux dimensions relativement modérées ;
- la photodiode peut être agencée dans une position radiale par rapport à la direction axiale X de la forme tubulaire ; de sorte que les dimensions peuvent être encore diminuées et l'intégration mécanique facilitée ;
- la cavité peut être un tube en aluminium ou un tube en plastique revêtu sur sa surface interne d'un dépôt métallique ; ainsi on dispose d'une cavité à la fois facile à fabriquer et présentant des bons coefficients de réflexion optique sur sa surface intérieure ;
- il n'y a pas de filtre interposé entre la source de lumière et la photodiode; de sorte que le second canal de mesure (référence) est particulièrement simple et ne nécessite pas de mise au point ou d'adaptation ;
- la source de lumière est commandée par une commande intermittente ; moyennant quoi on utilise une source lumineuse peu coûteuse et on limite la consommation électrique moyenne ;
- le détecteur peut être de type thermopile ou de type pyroélectrique ou encore de type photodiode travaillant dans le domaine infra-rouge; et avantageusement on utilise un seul détecteur de ce type de sorte que le coût du capteur est limité ;

- la consommation électrique cumulée du détecteur et de la photodiode est inférieure à 1 mA lorsqu'ils sont activés pour une mesure ; de sorte que d'un tel capteur peut être embarqué dans un appareil autonome ayant une autonomie électrique relativement modérée.

D'autres caractéristiques et avantages de l'invention apparaîtront au cours de la description suivante d'une de ses formes de réalisation, donnée à titre d'exemple non limitatif, en regard des dessins joints.

Sur les dessins :

- la figure 1 est une vue schématique du dispositif de mesure de concentration de gaz selon l'invention,
- les figures 2A et 2B représentent des diagrammes spectraux relatifs au dispositif de la figure 1,
- la figure 3 montre un schéma électrique de principe du dispositif de la figure 1, et
- la figure 4 montre des chronogrammes relatifs au dispositif de la figure 1.

Sur les différentes figures, les mêmes références désignent des éléments identiques ou similaires.

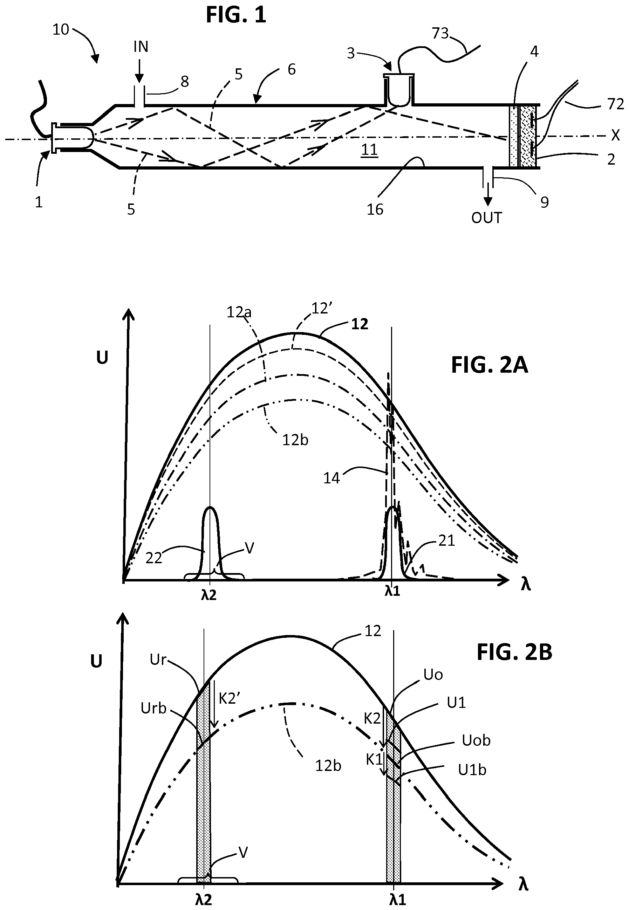

La figure 1 représente un dispositif **10** de mesure de concentration d'un gaz prédéterminé, en l'occurrence dans l'exemple illustré du dioxyde de carbone (CO2), présent dans l'air ambiant.

Toutefois, le principe présenté permet d'utiliser un tel dispositif pour mesurer la concentration de n'importe quel gaz prédéterminé ayant des propriétés d'absorption de rayons lumineux, en particulier des rayons lumineux dans le domaine infrarouge, ledit gaz prédéterminé étant inclus dans un ensemble gazeux donné **11.**

Le dispositif comprend tout d'abord une cavité **6** qui renferme l'ensemble gazeux **11** qui contient le gaz prédéterminé dans une concentration à mesurer ; dans l'exemple illustré, l'ensemble gazeux est l'air ambiant qui entre dans la cavité par une entrée **8** notée 'IN' été sort par une sortie **9** notée 'OUT' ; toutefois il pourrait n'y avoir qu'un seul orifice servant d'entrée et de sortie à la fois. Dans l'exemple illustré, la cavité prend la forme d'un tube droit, toutefois la forme pourrait être très différente, la génératrice du tube pourrait être courbe ; la cavité pourrait aussi se présenter sous la forme d'un tore ou de tout autre disposition géométrique. L'enveloppe de la cavité peut être en aluminium ou en matière plastique revêtue sur sa surface interne d'un dépôt métallique.

De plus, le dispositif **10** comprend une source de lumière **1,** plus généralement une source de rayonnements électromagnétiques incluant des rayons lumineux visibles et des rayons lumineux infrarouges, qui sont émis dans la cavité. Dans la présente description, le terme 'source de lumière' ou 'rayonnements lumineux' n'est donc pas limité au domaine du visible.

La source de lumière 1 est une lampe à filament classique ou une diode électroluminescente (Led).

La source de lumière émet des rayons lumineux **5** dans une gamme spectrale de base incluant du visible (0,4 à 0,8 $\mu$m) et de l'infra-rouge (0,8 $\mu$m à 500 $\mu$m).

Les rayons lumineux **5** se réfléchissent sur la surface interne **16** de la cavité et sont captées par différentes entités qui seront détaillées plus loin.

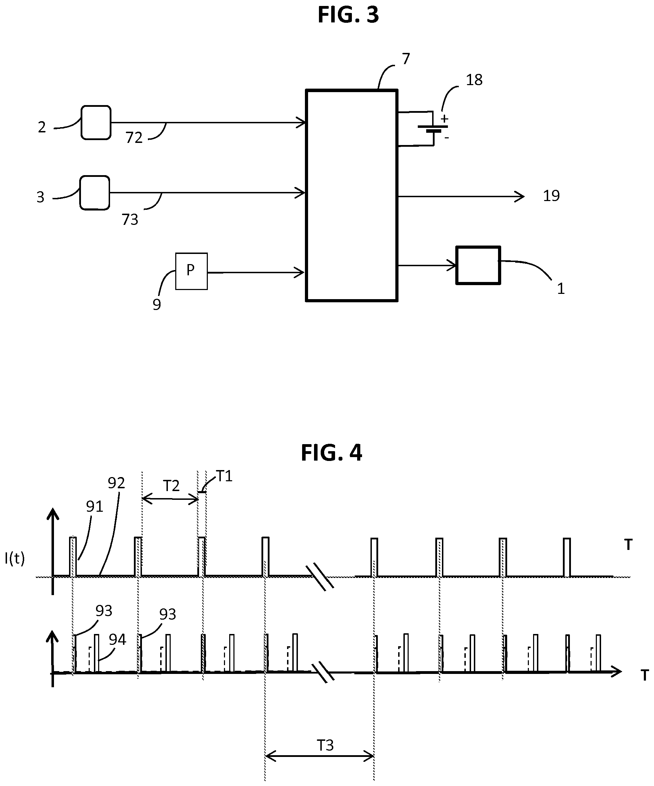

De plus, la lampe **1** est de préférence commandée par une commande intermittente telle que présentée à la Figure 4 qui sera commentée plus loin.

De plus, le dispositif **10** comprend un détecteur **2,** qui reçoit une partie des rayons lumineux **5** dans une première gamme spectrale prédéfinie correspondant à une forte absorption du gaz prédéterminé. Ce détecteur fournit un signal électrique proportionnel à la puissance lumineuse reçue par le détecteur, qui fait partie de ce qu'on peut aussi appeler un premier canal de mesure du capteur de CO2.

Le détecteur peut être un détecteur de type thermopile (comme par exemple le détecteur S60M TO5 de 'Dexter') ou de type pyroélectrique (tel que le LIE-201 de 'Infratec' par exemple). En alternative, on peut aussi utiliser un détecteur de type photodiode travaillant dans le domaine infra-rouge.

Avantageusement, on peut placer un filtre **4** en amont du détecteur, donc le filtre est interposé entre la source lumineuse **1** et le détecteur **2**. Dans ce cas, la puissance lumineuse reçue est restreinte à la portion spectrale qui traverse le filtre, illustrée par la caractéristique spectrale référencée **21** sur la figure 2A

En référence à la figure 2A, la source lumineuse 1 émet des rayonnements électromagnétiques dans un domaine spectral incluant le domaine infrarouge et le domaine **V** des rayonnements visibles ; plus précisément, cette émission est caractérisée par une courbe de densité de puissance spectrale **12** qui indique la répartition en termes de longueur d'onde de l'ensemble des rayonnements émis par la source lumineuse **1**. Dans le cas d'une lampe à filament, cette densité spectrale est plutôt de type large bande comme illustré, alors que pour une LED, cette courbe de densité spectrale sera moins large tout en englobant le domaine visible **V** et au moins une portion du domaine infrarouge proche.

Les caractéristiques d'émission de la lampe évoluent avec le temps ; on peut constater une augmentation de la puissance émise ou une diminution de la puissance émise ou encore les deux phénomènes l'un à la suite de l'autre. Le vieillissement de la lampe **1** entraîne un changement de la puissance émise, ce qui est illustré par la courbe **12a**, la puissance d'émission pouvant être réduite comme illustré (mais cela peut être aussi une augmentation). Ce n'est pas le seul facteur qui modifie la puissance reçue par le détecteur **2**. En effet, le dépôt de condensation, de poussière ou d'impuretés sur la surface intérieure de la cavité conduit à altérer le taux de réflexion des rayons lumineux **5** et par conséquent à diminuer la puissance transmise vers le détecteur 2, ce qui est illustré par la courbe référencée **12b.**

Il est donc nécessaire de prendre en compte ces altérations, qu'on peut aussi appeler 'dérives' (lentes ou rapides), afin que le calcul de concentration de CO2 ne soit pas entaché d'erreurs liée à ces dérives.

Il faut en outre tenir compte des dispersions de caractéristiques initiales des sources lumineuses : par exemple une autre lampe présentera une courbe de puissance spectrale **12'** différente.

À cet effet, le dispositif 10 comprend une **photodiode 3** configurée pour recevoir une partie des rayons lumineux dans une deuxième gamme spectrale prédéfinie correspondant à une faible absorption du gaz prédéterminé (ici le CO2), ce qui constitue un second canal de mesure. Il faut noter que les rayons lumineux sont reçus directement par la photodiode **3,** sans filtre interposé entre la source **1** et la photodiode.

Plus précisément, la photodiode **3** est principalement sensible aux longueurs d'onde dans le domaine visible $(0,4$ à $0,8\ \mu m)$; ceci peut également être illustré par la caractéristique spectrale référencée **22,** de type passe-bande centré sur $\lambda$**2**. Le CO2 n'absorbe pratiquement pas les rayonnements lumineux visibles et par conséquent la quantité de lumière reçue par la photodiode 3 ne dépend pas de la concentration de CO2 dans la cavité. La quantité de lumière ou puissance lumineuse reçue par la photodiode 3 dépend en revanche de la puissance émise par la lampe 1 et des coefficients de réflexion sur les parois internes 16 de la cavité.

Il est à noter que ce type de photodiode sensible aux rayonnements visibles est très peu coûteux car il utilise essentiellement un coeur en silicium.

La caractéristique d'atténuation des rayons par le CO2 selon la longueur d'onde est illustrée par la courbe en pointillés référencée **14** sur la figure 2A. Avantageusement, le filtre **4** est de type passe-bande centré sur $\lambda$1, ce qui coïncide avec la zone d'atténuation maximum de la courbe **14**. Pour le CO2, la longueur d'onde $\lambda$**1** est choisie tel que $\lambda 1 = 4{,}26\ \mu m$ (micromètres).

En référence à la figure 2B, la puissance reçue par la photodiode 3 (second canal) est représentée par la quantité de référence **Ur** lorsque le dispositif de mesure est dans un état neuf, la grandeur **Ur** étant connue par le test de fin de fabrication du capteur de CO2 ; en fonction du temps et des altérations qui peuvent intervenir, la quantité de référence **Ur** devient **Urb,** ce qui prend en compte non seulement la diminution de puissance de la lampe mais aussi toutes les dérives qui peuvent intervenir concernant la lampe et la cavité.

Avantageusement selon l'invention, les quantités de lumière mesurée sur le premier et le deuxième canal sont en fait établies par différence entre un état actif pour lequel la lampe est allumée et un état repos pour laquelle la lampe est éteinte. Plus précisément, pour toutes les valeurs U ou $U_x$ exprimées dans le paragraphe précédent ou les paragraphes suivants, il faut comprendre **U = U$_{ON}$ - U$_{OFF}$.** Ceci permet d'éliminer une éventuelle composante continue, due à l'éclairage ambiant ou des parasites lumineux.

S'agissant du premier canal de mesure relatif au détecteur 2, la quantité lumineuse reçue par le détecteur **2** qui correspondrait à une concentration en CO2 égal à zéro est référencé **Uo** ; la quantité lumineuse reçue pour une concentration en CO2 à mesurer est référencée **U1**, plus faible que **Uo** du fait de l'atténuation.

Avec les effets du vieillissement, représentée par la courbe 12b, la quantité lumineuse reçue par le détecteur **2** qui correspondrait à une concentration en CO2 égal à zéro est référencé **Uob ;** la quantité lumineuse reçue pour la concentration en CO2 à mesurer est référencée **U1b,** plus faible que **Uob** du fait de l'atténuation.

U1b/Uob = U1/Uo est le facteur d'atténuation **K1** sur le canal $\lambda$1, dû à l'effet atténuateur du CO2.

Par ailleurs, Uob/Uo et Urb/Ur sont des facteurs de dérive lié au vieillissement et autres phénomène susmentionnés, ils sont notés respectivement K2 et K2'. Ils sont liés entre eux par une fonction **F** prédéfinie, par exemple K2'=F(K2).

$$K1 = \frac{U_1 b}{Uob} = \frac{U_1 b}{Uo}.\mathbf{G}(Urb/Ur) \quad (Eq.\ 1),$$

la fonction **G** rassemblant les facteurs de correction, en particulier liés à **K2** et **K2'**.

Dans une version simplifiée, on pourrait approximer la fonction F par la fonction identité et ainsi obtenir une expression simplifiée de l'équation précédente :

$$K1 = \frac{U_1 b}{Uob} = \frac{U_1 b.Ur}{Uob.Urb} \quad (Eq.\ 1a)$$

On ajoute également le cas échéant des corrections relatives à la température et à la pression ambiante (voir plus loin).

Sachant que Uo et Ur sont connus de la calibration initiale du capteur, et que Urb et U1b correspondent aux mesures de puissance lumineuse mesurées (en différentiel ON/OFF pour éliminer la composante continue, éclairage ambiant) sur le détecteur 2 et la photodiode 3, on utilise l'équation Eq. 1 pour déterminer le facteur d'atténuation lié à la présence de CO2 ; on se reporte ensuite à une courbe de type abaque qui relie le facteur d'atténuation à la concentration en CO2. Cette abaque peut prendre en compte en outre la pression et la température ambiante.

Pour pouvoir notamment mettre en oeuvre les opérations susmentionnées, le dispositif 10 comprend une unité de commande **7** alimentée par une alimentation électrique **18,** notamment sous forme d'une batterie ou pile classique ou rechargeable.

En référence à la figure 3, le détecteur 2 est relié à l'unité de commande par une liaison électrique **72,** et la photodiode **3** est reliée à l'unité de commande par une liaison électrique **73.**

De plus un capteur de pression 9 et un capteur de température peuvent être prévus pour que l'unité de commande **7** puisse acquérir l'information de la pression atmosphérique ambiante et la température ambiante. En outre, le capteur de température (non représenté) peut être embarqué sur la carte de l'unité de commande **7.**

En référence à la figure 4, l'unité de commande **7** pilote la source lumineuse **1** selon une séquence prédéterminée.

Plus précisément, dans l'exemple illustré, la lampe est commandée à l'état ON **91** pendant un temps **T1** de 200 ms, puis est commandée à l'état OFF **92** pendant le temps **T2** de 600 ms, séquence qui est répétée dans l'exemple illustré quatre fois, le nombre de répétitions pouvant être quelconque.

Les mesures **93,94** correspondantes de puissance lumineuse reçue sont effectuées à chaque répétition d'une part pendant la période T1 à l'état ON ($U_{ON}$ comme déjà mentionné plus haut, ref. 93) et d'autre part pendant la période T2 à l'état OFF (notation $U_{OFF}$ déjà mentionnée plus haut, ref. 94). Comme déjà indiqué plus haut, c'est la différence de puissance entre l'état ON et l'état OFF qui est pris en compte comme quantité de lumière reçue à la fois par le détecteur **2** et par la photodiode **3.**

La séquence de mesure est répétée après un laps de temps d'inactivité de durée **T3** prédéterminée, cette durée pouvant être choisie dans une plage de valeurs entre 10 minutes et 30 minutes ; la consommation moyenne du capteur de CO2 est par conséquent très faible.

Il faut noter que la consommation électrique de la photodiode est de l'ordre de 10 uA (microampères); de fait elle est très inférieure à la consommation électrique du détecteur de type thermopile qui lui consomme un peu moins de 1 mA. Ainsi, la consommation électrique cumulée du détecteur et de la photodiode est inférieure à 1 mA lorsqu'ils sont activés pour une mesure.

En outre, avantageusement, il peut être prévu une transmission des données recueillies vers un système utilisateur, par une liaison sans fil **19.**

Après l'assemblage initial du capteur, on procède à une étape de calibration au cours de laquelle on détermine la caractéristique de la courbe de puissance spectrale de la lampe. On peut par exemple mesurer la grandeur **Ur.** On peut aussi mesurer la grandeur **U1** pour une concentration en CO2 connue, et retrouver ainsi la grandeur **Uo** utilisés dans l'équation 'Eq.1' ci-dessus.

Avantageusement, un tel capteur de concentration de CO2, peu encombrant et peu coûteux, peut être intégré dans un certain d'objets d'utilisation courante, comme par exemple un pèse-personne électronique, un moniteur d'activité personnelle, etc...

**Revendications**

**1.** Dispositif de mesure de la concentration d'un gaz prédéterminé, comprenant :

- une cavité (6) renfermant un ensemble gazeux contenant du gaz prédéterminé dans une concentration à mesurer,
- une source de lumière (1), de type lampe à filament ou diode électroluminescente, apte à émettre dans la cavité des rayons lumineux (5) dans une gamme spectrale de base incluant du visible et de l'infra-rouge,
- un détecteur infrarouge (2), configuré pour recevoir une partie des rayons lumineux dans une première gamme spectrale prédéfinie correspondant à une forte absorption du gaz prédéterminé,
- une photodiode (3) configurée pour recevoir une partie des rayons lumineux dans une deuxième gamme spectrale prédéfinie correspondant à une faible absorption du gaz prédéterminé,
- une unité de commande (7) configurée pour calculer la concentration du gaz prédéterminé en comparant des signaux électriques représentatifs respectivement de la puissance lumineuse reçue par le détecteur infrarouge (2) et de la puissance lumineuse reçue par la photodiode (3), **caractérisé en ce que** la photodiode (3) travaille dans le domaine visible, principalement sensible aux longueurs d'onde comprises entre 0,4 et 0,8 $\mu$m.

**2.** Dispositif selon la revendication 1, dans lequel le gaz prédéterminé est le dioxyde de carbone CO2 et l'ensemble gazeux est l'air ambiant.

**3.** Dispositif selon la revendication 2, comprenant un filtre (4) interposé entre la source (1) et le détecteur (2), ledit filtre étant centré sur la longueur d'onde $\lambda 1$ = 4,26 $\mu$m.

**4.** Dispositif selon l'une des revendications 1 à 3, dans lequel la cavité a une forme générale tubulaire, ayant de préférence un diamètre inférieure à 10 mm et une longueur de préférence inférieure 10 cm, les rayons étant dirigés substantiellement selon une direction axiale (X) de la forme tubulaire.

**5.** Dispositif selon la revendication 4, dans lequel la photodiode est agencée dans une position radiale par rapport à la direction axiale (X) de la forme tubulaire.

**6.** Dispositif selon la revendication 4, dans lequel la cavité est un tube en aluminium ou un tube en plastique revêtu sur sa surface interne d'un dépôt métallique.

**7.** Dispositif selon l'une des revendications 1 à 6, dans lequel il n'y a pas de filtre interposé entre la source de lumière (1) et la photodiode (3).

**8.** Dispositif selon l'une des revendications 1 à 7, dans lequel la source de lumière (1) est commandée par une commande intermittente.

**9.** Dispositif selon l'une des revendications 1 à 8, dans lequel le détecteur (2) est de type thermopile ou de type pyroélectrique ou encore de type photodiode travaillant dans le domaine infra-rouge.

**10.** Dispositif selon la revendication 9, comprenant un seul détecteur dudit type.

**11.** Dispositif selon l'une des revendications 1 à 10, dans lequel la consommation électrique cumulée du détecteur (2) et de la photodiode (3) est inférieure à 1 mA lorsqu'ils sont activés pour une mesure.

**Patentansprüche**

**1.** Vorrichtung zum Messen der Konzentration eines vorbestimmten Gases, umfassend:

- einen Hohlraum (6), der eine Gasanordnung enthält, die vorbestimmtes Gas in einer zu messenden Konzentration enthält,
- eine Lichtquelle (1) vom Typ der Glühfadenlampe oder der Leuchtdiode, die geeignet ist, Lichtstrahlen (5) in einem Grundspektralbereich, der Infrarotstrahlung oder sichtbares Licht aufweist, in den Hohlraum abzugeben,
- einen Detektor (2), der konfiguriert ist, um einen Teil der Lichtstrahlen in einem ersten vorbestimmten Spektralbereich zu empfangen, der einer starken Absorption des vorbestimmten Gases entspricht,
- eine Photodiode (3), die konfiguriert ist, um einen Teil der Lichtstrahlen in einem zweiten vorbestimmten Spektralbereich zu empfangen, der einer schwachen Absorption des vorbestimmten Gases entspricht,
- eine Steuereinheit (7), die konfiguriert ist, um die Konzentration des vorbestimmten Gases zu berechnen, indem elektrische Signale verglichen werden, die jeweils für die Lichtleistung, die von dem Infrarotdetektor (2) empfangen wird, und die Lichtleistung, die von der Photodiode (3) empfangen wird, repräsentativ sind,

**dadurch gekennzeichnet, dass** die Photodiode (3) im sichtbaren Bereich arbeitet, die hauptsächlich für Wellenlängen zwischen 0,4 und 0,8 $\mu$m empfindlich ist.

**2.** Vorrichtung nach Anspruch 1, wobei das vorbestimmte Gas Kohlendioxid CO2 und die Gasanordnung die Umgebungsluft ist.

**3.** Vorrichtung nach Anspruch 2, umfassend einen Filter (4), der zwischen der Quelle (1) und dem Detektor (2) angeordnet ist, wobei der Filter auf der Wellenlänge $\lambda 1$ = 4,26 $\mu$m zentriert ist.

**4.** Vorrichtung nach einem der Ansprüche 1 bis 3, wobei der Hohlraum eine allgemein röhrenförmige Form aufweist, die vorzugsweise einen Durchmesser von unter 10 mm und eine Länge von vorzugsweise unter 10 cm aufweist, wobei die Strahlen im Wesentlichen entlang einer axialen Richtung (X) der röhrenförmigen Form gerichtet sind.

**5.** Vorrichtung nach Anspruch 4, wobei die Photodiode in einer radialen Position in Bezug auf die axiale Richtung (X) der röhrenförmigen Form angeordnet ist.

**6.** Vorrichtung nach Anspruch 4, wobei der Hohlraum ein Aluminiumrohr oder ein Kunststoffrohr ist, das auf seiner Innenfläche mit einer metallischen Abscheidung beschichtet ist.

**7.** Vorrichtung nach einem der Ansprüche 1 bis 6, wobei kein Filter vorhanden ist, der zwischen der Lichtquelle (1) und der Photodiode (3) angeordnet ist.

**8.** Vorrichtung nach einem der Ansprüche 1 bis 7, wobei die Lichtquelle (1) durch eine intermittierende Steuerung gesteuert wird.

**9.** Vorrichtung nach einem der Ansprüche 1 bis 8, wobei der Detektor (2) vom Typ des Thermopile-Detektors oder vom Typ des Pyrodetektors oder auch vom Typ der Photodiode ist, die im Infrarotspektrum arbeitet.

**10.** Vorrichtung nach Anspruch 9, die einen einzigen Detektor des Typs aufweist.

**11.** Vorrichtung nach einem der Ansprüche 1 bis 10, wobei der kumulierte Stromverbrauch des Detektors (2) und der Photodiode (3) niedriger als 1 mA ist, wenn sie für eine Messung aktiviert werden.

**Claims**

**1.** Device for measuring the concentration of a predetermined gas, comprising:

- a cavity (6) holding a gas mixture containing the predetermined gas in a concentration to be measured,
- a light source (1) of the filament lamp type or electroluminescent diode type, capable of emitting, within the cavity, light rays (5) within a basic spectral range including visible and infrared,
- a detector (2) configured to receive a portion of the light rays within a first predefined spectral range corresponding to high absorption of the predetermined gas,
- a photodiode (3) configured to receive a portion of the light rays within a second predetermined spectral range corresponding to low absorption of the predetermined gas,
- a control unit (7) configured to calculate the concentration of the predetermined gas by comparing electrical signals respectively representative of the radiant power received by the detector (2) and of the radiant power received by the photodiode (3), **characterized in that** the photodiode (3) operates in the visible range, mainly sensitive to wavelengths comprised between 0,4 and 0,8 $\mu$m.

**2.** Device according to claim 1, wherein the predetermined gas is carbon dioxide $CO_2$ and the gas mixture is ambient air.

**3.** Device according to claim 2, comprising a filter (4) interposed between the source (1) and the detector (2), said filter being centered on the wavelength $\lambda 1 = 4.26$ $\mu$m.

**4.** Device according to one of claims 1 to 3, wherein the cavity has a generally tubular shape, preferably with a diameter less than 10 mm and a length preferably less than 10 cm, the rays being directed substantially in an axial direction (X) of the tubular shape.

**5.** Device according to claim 4, wherein the photodiode is arranged in a radial position relative to the axial direction (X) of the tubular shape.

**6.** Device according to claim 4, wherein the cavity is an aluminum tube or a plastic tube coated with a metal deposit on its inner surface.

**7.** Device according to one of claims 1 to 6, wherein there is no filter interposed between the light source (1) and the photodiode (3).

8. Device according to one of claims 1 to 7, wherein the light source (1) is a filament lamp or a light emitting diode LED, preferably controlled by an intermittent control.

9. Device according to one of claims 1 to 8, wherein the type of detector (2) is thermopile or pyroelectric or a photodiode operating in the infrared range.

10. Device according to claim 9, comprising a single detector of said type.

11. Device according to one of claims 1 to 10, wherein the cumulative power consumption of the detector (2) and photodiode (3) is less than 1 mA when they are activated for a measurement.

## FIG. 1

## FIG. 2A

## FIG. 2B

# FIG. 3

# FIG. 4

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 8003944 B

- US 20050173635 A1